(19) Europäisches Patentamt European Patent Office Office européen des brevets

(11) **EP 2 038 010 B1**

(12) **EUROPEAN PATENT SPECIFICATION**

(45) Date of publication and mention
of the grant of the patent:
**04.09.2013 Bulletin 2013/36**

(21) Application number: **07858866.2**

(22) Date of filing: **06.07.2007**

(51) Int Cl.:
**A61N 5/10** (2006.01)

(86) International application number:
**PCT/IB2007/003392**

(87) International publication number:
**WO 2008/035213 (27.03.2008 Gazette 2008/13)**

(54) **SPATIALLY-VARIANT NORMAL TISSUE OBJECTIVE FOR RADIOTHERAPY**

RÄUMLICH VARIABLER NORMALGEWEBE-ZIELBEREICH FÜR DIE STRAHLENTHERAPIE

OBJECTIF DE TISSU NORMAL VARIANT DANS L'ESPACE POUR UNE RADIOTHÉRAPIE

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **06.07.2006 US 819255 P
25.01.2007 US 698617**

(43) Date of publication of application:
**25.03.2009 Bulletin 2009/13**

(73) Proprietor: **Varian Medical Systems International
AG
6303 Zug (CH)**

(72) Inventors:
• **ALAKUIJALA, Jyrki, Antero
FIN-02180 Espoo (FI)**
• **PESOLA, Katja, Marika
FIN-01650 Vantaa (FI)**

(74) Representative: **Viering, Jentschura & Partner
Grillparzerstrasse 14
81675 München (DE)**

(56) References cited:
**US-A- 3 987 281       US-A1- 2006 067 469
US-B1- 6 222 905**

• **SPIROU SPIRIDON V ET AL: "A gradient inverse
planning algorithm with dose-volume
constraints" MEDICAL PHYSICS, AIP, MELVILLE,
NY, US, vol. 25, no. 3, March 1998 (1998-03), pages
321-333, XP012010426 ISSN: 0094-2405**

**Description**

**FIELD**

This application relates generally to radiation systems and methods, and more specifically, to systems and methods for controlling radiation dose in normal tissue in a radiation procedure.

**BACKGROUND**

Radiation treatment procedures are commonly performed to treat various medical conditions. In a radiation treatment procedure, a radiation beam is directed towards a target tissue region. The radiation beam may be collimated using a collimator, such as a multi-leaf collimator, to thereby provide a desirable shape for the radiation beam. In some cases, such feature allows a shape of the radiation beam to conform with the target tissue region without injuring surrounding normal/healthy tissue.

Sometimes, in a radiation treatment procedure, a plurality of treatment sessions may be performed. In each treatment session, a radiation source may be placed at a prescribed gantry angle to thereby deliver radiation beam towards a target tissue from a certain angle. As a result of delivering radiation towards the target tissue from a plurality of different angles, a sufficient radiation dose may be delivered to the target tissue to thereby treat the target tissue, while surrounding healthy tissue may be protected. The SI unit of energy for absorbed dose of radiation is Gray (Gy). One Gray is defined as is the absorption of one joule of radiation energy by one kilogram of matter.

It has been known that delivering radiation towards a target tissue from different angles may result in creation of hotspot(s) at healthy tissue(s). Currently, hotspots are attempted to be minimized or reduced by drawing or inputting artificial structures representing normal (healthy) tissues into a computer program. Thereafter, the computer program imposes a constraint (representing a limit on radiation dose to be received by the healthy tissue) on the drawn artificial structures, and determines a treatment plan based on the imposed constraint. However, the technique of drawing artificial structures to represent normal tissues is a laborious and time consuming process. The result may also not be desirable because the drawing of artificial structures may only move the hot spot to a new location. Sometimes, after a treatment plan has been determined, the computer program performs a simulation to determine an effect of radiation that is to be delivered in accordance with the treatment plan on the target tissue and adjacent healthy tissues. If hotspot(s) still exists, the user will have to revise the input model to modify the previously drawn artificial structures and/or to input additional artificial structure(s) to address the hotspot(s). As such, existing techniques for fluence optimization is an iterative process that is laborious and time consuming.

US 2006/067469 A1 discloses a flexible radiation treatment planning system configured to enable the user to utilize both forward planning and inverse planning techniques. US 3,987,281 discloses a radiation treatment planning system setting spatially variable constraints on healthy tissue.

**SUMMARY**

The present invention provides a method for use to determine or evaluate a radiation treatment plan and a system to perform such a method having the features described in the independent claims. Preferred embodiments of the present invention are described in the dependent claims.

In accordance with some embodiments, a method for use to determine or evaluate a radiation treatment plan includes determining a spatially variable constraint, and imposing the spatially variable constraint on a healthy tissue.

In accordance with other embodiments, a system for use to determine or evaluate a radiation treatment plan includes a processor configured for determining a spatially variable constraint, and imposing the spatially variable constraint on a healthy tissue.

In accordance with other embodiments, a method for use to determine or evaluate a radiation treatment plan includes determining a first position of a first healthy tissue, imposing a first constraint on the first healthy tissue based on the determined first position, determining a second position of a second healthy tissue, and imposing a second constraint on the second healthy tissue based on the determined second position, wherein the first constraint and the second constraint have different values.

In accordance with other embodiments, a system for use to determine or evaluate a radiation treatment plan includes a processor configured for determining a first position of a first healthy tissue, imposing a first constraint on the first healthy tissue based on the determined first position, determining a second position of a second healthy tissue, and imposing a second constraint on the second healthy tissue based on the determined second position, wherein the first constraint and the second constraint have different values.

In accordance with other embodiments, a radiation process includes determining a treatment plan using a spatially variable constraint on healthy tissue, and performing a radiation procedure based on the determined treatment plan.

In accordance with other embodiments, a radiation system includes a processor configured for determining a treatment plan using a spatially variable constraint on healthy tissue, and a radiation machine for performing a radiation procedure based on the determined treatment plan.

Other aspects and features will be evident from reading the following detailed description of the embodiments.

## BRIEF DESCRIPTION OF THE DRAWINGS

The drawings illustrate the design and utility of embodiments, in which similar elements are referred to by common reference numerals. In order to better appreciate how advantages and objects of the embodiments are obtained, a more particular description of the embodiments will be illustrated in the accompanying drawings.

**FIG. 1** illustrates a system for performing a radiation procedure in accordance with a treatment plan determined in accordance with some embodiments;

**FIG. 2** illustrates an example of a normal tissue constraint curve in accordance with some embodiments;

**FIGS. 3A-3B** illustrate an example of determining a normal tissue constraint based on a plurality of target tissues; and

**FIG. 4** illustrates a block diagram of a computer system that can be used to perform various functions described herein in accordance with some embodiments.

## DETAILED DESCRIPTION OF THE EMBODIMENTS

Various embodiments are described hereinafter with reference to the figures. It should be noted that the figures are not drawn to scale and elements of similar structures or functions are represented by like reference numerals throughout the figures. It should also be noted that the figures are only intended to facilitate the description of embodiments. They are not intended as an exhaustive description of the invention or as a limitation on the scope of the invention. In addition, an aspect described in conjunction with a particular embodiment is not necessarily limited to that embodiment and can be practiced in any other embodiments.

Treatment System

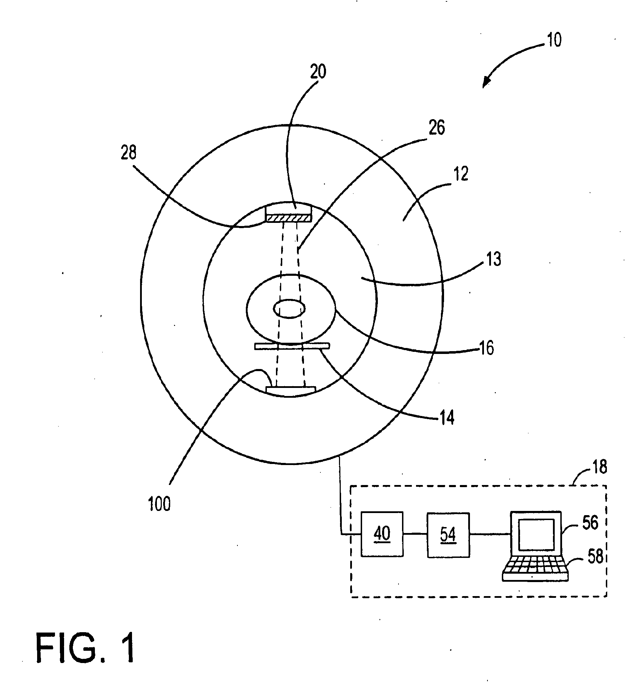

**FIG. 1** illustrates a radiation system 10 in accordance with some embodiments. The system 10 includes a gantry 12 having an opening (or bore) 13, a patient support 14 for supporting a patient 16, and a control system 18 for controlling an operation of the gantry 12. The system 10 also includes a radiation source 20 that projects a beam 26 of radiation towards the patient 16 while the patient 16 is positioned at least partially within the opening 13. The radiation source 20 can be configured to generate a cone beam, a fan beam, or other types of radiation beams in different embodiments. The system 10 further includes a collimator system 28 secured to the radiation source 20 for controlling a delivery of the radiation beam 26. The collimator system 28 can be, for example, a multi-leaf collimator, or other beam adjuster known in the art. The system 10 further includes an imager 100 located opposite from the radiation source 20. Before or during a treatment procedure, the imager 100 may be used to obtain an image of a target tissue region. The image may be used to determine tissue shape, geometry, location, and/or dosage information. In other embodiments, the imager 100 is optional, and the system 10 does not include the imager 100.

In the illustrated embodiments, the radiation source 20 is a treatment radiation source for providing treatment energy. In other embodiments, the radiation source 20 may be a diagnostic radiation source for providing diagnostic energy (e.g., energy that is suitable for generating an image). In further embodiments, the radiation source 20 can be configured to selectively provide treatment energy and diagnostic energy. In some embodiments, the treatment energy is generally those energies of 160 kilo-electron-volts (keV) or greater, and more typically 1 mega-electron-volts (MeV) or greater, and diagnostic energy is generally those energies below the high energy range, and more typically below 160 keV. In other embodiments, the treatment energy and the diagnostic energy can have other energy levels, and refer to energies that are used for treatment and diagnostic purposes, respectively. In some embodiments, the radiation source 20 is able to generate X-ray radiation at a plurality of photon energy levels within a range anywhere between approximately 10 keV and approximately 20 MeV. Radiation sources capable of generating X-ray radiation at different energy levels are described in U.S. Patent Application Serial No. 10/033,327, entitled "RADIOTHERAPY APPARATUS EQUIPPED WITH AN ARTICULABLE GANTRY FOR POSITIONING AN IMAGING UNIT," filed on November 2, 2001, and U.S. Patent Application Serial No. 10/687,573, entitled "MULTI-ENERGY X-RAY SOURCE," filed on October 15, 2003.

In the illustrated embodiments, the control system 18 includes a processor 54, such as a computer processor, coupled to a control 40. The control system 18 may also include a monitor 56 for displaying data and an input device 58, such as a keyboard or a mouse, for inputting data. In the illustrated embodiments, the gantry 12 is rotatable about the patient 16, and during an imaging procedure, the gantry 12 rotates about the patient 16. The operation of the radiation source 20 and the gantry 12, are controlled by the control 40, which provides power and timing signals to the radiation source 20, and controls a rotational speed and position of the gantry 12, based on signals received from the processor 54. Although the control 40 is shown as a separate component from the gantry 12 and the processor 54, in alternative embodiments, the control 40 can be a part of the gantry 12 or the processor 54. In some embodiments, the operation of the collimator system 28 is also controlled by the processor 54. In further embodiments, the control 40 also controls a position of the patient support 14. For example, the control 40 may cause the patient support 14 to translate relative to the opening 13.

In the illustrated embodiments, the processor 54 is configured to process a treatment plan, and operate the gantry 12 and/or the collimator system 28 in accordance with the treatment plan, wherein the treatment plan may be determined using techniques described herein. For example, in some embodiments, the processor 54 may be configured (e.g., programmed) in accordance with the treatment plan to position one or more leafs of the collimator system 28 to thereby allow a beam having a desired shape to be created. In other embodiments, the processor 54 may be configured to position one or more leafs of the collimator system 28 to track a moving target tissue. In further embodiments, the processor 54 may be configured in accordance with the treatment plan to position one or more leafs of the collimator system 28 to perform an Intensity Modulated Radiation Therapy (IMRT) on a target tissue, wherein a portion of the target tissue is radiated to receive more radiation dose than another portion of the target tissue. Also, in further embodiments, the processor 54 may be configured in accordance with the treatment plan to track a moving target tissue and simultaneously perform IMRT on the target tissue. At least a portion of the treatment plan may be determined in accordance with embodiments of technique described herein.

It should be noted that the radiation system 10 should not be limited to the configuration described previously, and that the radiation system 10 can also have other configurations in other embodiments. For example, in other embodiments, instead of a ring-configuration, the radiation system 10 can have a C-arm configuration. Also, in other embodiments, the radiation system 10 can include an arm to which the radiation source 20 is secured. In further embodiments, the radiation system 10 can have configurations that are known in the art of radiation systems.

Normal Tissue Constraint

In accordance with some embodiments, a treatment plan may be determined using Normal Tissue Constraint (NTC). NTC represents an optimization constraint for a body part (e.g., normal/healthy tissue) that does not include a Planning Target Volume (PTV). PTV is defined as Clinical Target Volume (CTV), e.g., abnormal/unhealthy tissue, plus a prescribed margin, e.g., 5 mm, to account for positional uncertainties. The NTC constraint may be provided to take into account the decrease in a dose level as the distance from the PTV(s) is increased. In accordance with some embodiments, a shape of NTC is determined by one or more of the following parameters: distance from PTV border ($X_{start}$) [mm], normalized level of start dose ($f_o$), normalized level of end dose (f-), and fall-off factor (k) [1/mm]. In particular, the shape of NTC as a function f(x) of a distance x [mm] from PTV border is calculated according to the following formula:

$$f(x) = \begin{cases} f_o e^{-k(x - x_{start})} + f_\infty (1 - e^{-k(x - x_{start})}), x \geq x_{start} \\ f_o, x \leq x_{start} \end{cases}$$

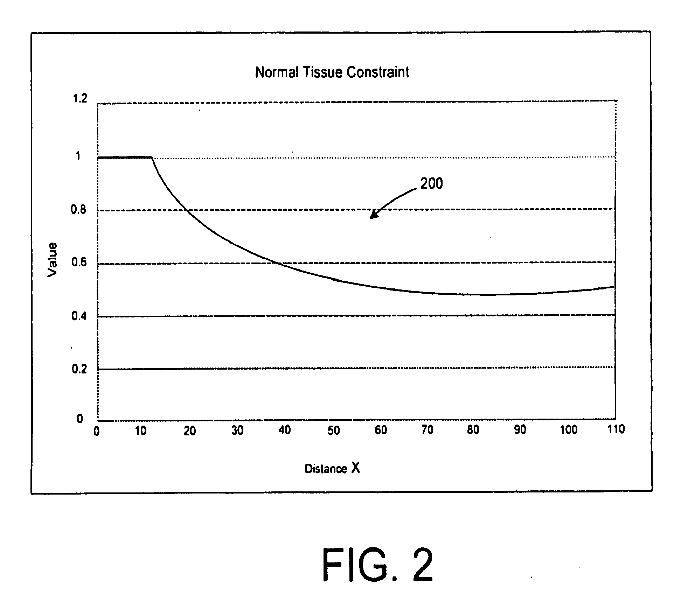

FIG. 2 illustrates an example of a NTC curve calculated using the following parameter values: $X_{start}$ = 10 mm, $f_o$ = 1.0, f- = 0.5, and k = 0.05 1/mm. In other embodiments, the NTC function f(x) may be calculated using other parameter values. As shown in **FIG. 2,** the prescribed constraint value by the NTC curve is 1.0, for x = 0 to 10 mm (which is the distance from the PTV border), and the constraint value decreases exponentially as a function of x, for x > 10 mm. As shown in the example, for a healthy tissue that is located at 40 mm away from the PTV border, the NTC value is approximately 0.6. For a healthy tissue that is located at 100 mm away from the PTV border, the NTC value is approximately 0.5. As such, the prescribed constraint by the NTC on healthy tissue varies spatially, in which healthy tissue closer to the PTV is imposed with a higher constraint value, and healthy tissue further away from the PTV is imposed with a lower constraint value. Such technique allows behavior of radiation interaction be modeled more accurately, which in turn, allows a treatment plan to be determined with higher accuracy and efficiency. As such, the above technique using NTC is advantageous over existing techniques in which a same constraint (e.g., a prescribed minimum radiation dose) is used for all healthy tissue regardless of its distance from the PTV.

In some embodiments, after NTC has been determined, it is inputted as a constraint for healthy tissue. A computer simulation may be performed based on the NTC to predict an effect of a set of radiation beams on tissue regions, which include target and healthy tissues. In some cases, parameters of the NTC function may be adjusted. Once a set of desired radiation beams have been determined, it is then incorporated as a treatment plan.

In the above example, the NTC curve is normalized such that the highest NTC value is equal to 1.0. In other embodiments, the NTC curve may not be normalized. For example, the normalized NTC curve may be multiplied by a factor F such that the highest value on the NTC curve is F. Also, in other embodiments, the NTC may be represented by other functions. For example, in other embodiments, instead of decreasing exponentially, the NTC function f(x) may decrease linearly as a function of x. As another example, instead of having the flat portion shown in FIG. 2, the NTC curve may have a decreasing profile starting from x = 0 mm. In further embodiments, instead of representing a distance from a PTV border, the variable x may represent a distance from a CTV border, or a distance from a centroid of a CTV.

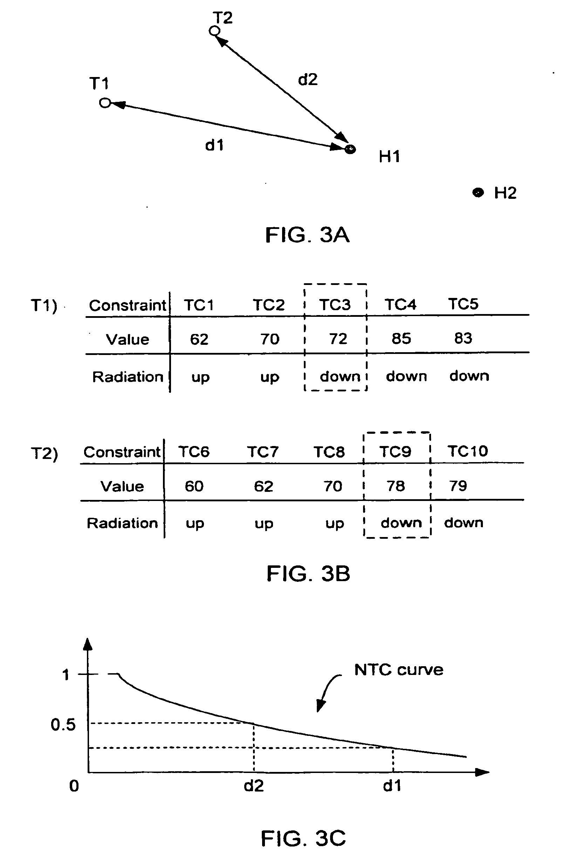

In some embodiments, a NTC for a particular healthy tissue may be determined based on target constraints associated with a plurality of respective target tissues. **FIGS. 3A-3C** illustrate an example of determining a NTC for a healthy tissue H1 that is located at a first distance d1 from a first target tissue T1, and at a second distance d2 from a second target tissue T2. The target tissue points T1, T2 and the healthy tissue point H1 may be inputted into a computer program as a mathematical model, where the target tissue points T1, T2 represent points/regions in or on respective CTVs (or in or on respective PTVs), and H1 represents a point/region in a healthy tissue. As such, as used in this specification, the term "target tissue" is not limited to real tissue, and may refer to artificial target tissue in a computer model. Similarly, as used in this specification, the term "healthy tissue" or "normal tissue" is not limited to real tissue, and may refer to artificial healthy/normal tissue in a computer model. In some embodiments, a density of target tissue points T in the mathematical model may be higher than that of healthy tissue points H (e.g., H1, H2, etc.). For example, the spacing between adjacent target tissue points T may be a value between 2 mm and 3 mm, inclusive, while the spacing between adjacent healthy tissue points H may be a value between 4 mm and 5 mm, inclusive. Alternatively, the density of target tissue points T and healthy tissue points H may be the same.

In accordance with some embodiments, a first set of target constraints (TC1-TC5 in the example) may be inputted for target tissue T1, and a second set of target constraints (T6-T10 in the example) may be inputted for target tissue T2 **(FIG. 3B).** In the examples, target constraints TC1-TC5 have respective values 62 Gy, 70 Gy, 72 Gy, 85 Gy, and 83 Gy, and target constraints TC6-TC10 have respective values 60 Gy, 62 Gy, 70 Gy, 78 Gy, and 79 Gy. As shown in the example, target constraints TC3, TC4, and TC5 for target tissue T1 are upper constraints because they have the effect of pushing down a radiation dose, while target constraints TC1 and TC2 are lower constraints because they have the effect of pushing up a radiation dose. Also in the example, target constraints TC9 and TC10 are for target tissue T2 are upper constraints, and TC6, TC7, and TC8 are lower constraints. In the illustrated embodiments, a constraint may represent a radiation dose or a variable that affect a radiation dose. In some embodiments, the constraint values TC may be determined from clinical data that is known in the art.

It should be noted that the number of target constraints for each target tissue is not limited to that shown, and that more or less than five target constraints (e.g., one constraint) may be inputted for a target tissue in other embodiments. Also, the constraint values may have other values in other examples. Further, in other embodiments, the number of target constraints inputted for a first target tissue may be different from the number of target constraints inputted for a second target tissue.

In other embodiments, the number of upper constraint(s) and lower constraint(s) for a target tissue may be different from the examples illustrated. For example, in some embodiments, one lower constraint may be inputted for a target tissue. This ensures that the target tissue will receive a radiation dose. In other embodiments, additional lower constraint(s) may be inputted for the target tissue, thereby allowing the radiation dose extremeties within a target to be further adjusted. Also, in some embodiments, the number of upper constraints for a target tissue may be zero. Alternatively, one or more upper constraints may be inputted for the target tissue, thereby preventing a radiation dose at the target tissue to be too high to avoid toxicity effect.

Also, in some embodiments, the constraint values may be entered into a computer program through a user interface. For example, a graphical user interface (such as that provided by Eclipse™ Treatment Planning System (Varian Medical Systems, Inc., Palo Alto, CA, USA), may be used to input a number of constraint(s) for a target tissue, one or more constraint values, and to specify whether a constraint is an upper constraint or a lower constraint.

Next, the upper target constraint with the lowest value is selected for each of the target tissues T1, T2. In the example, this corresponds to target constraint TC3 = 72 Gy being selected for target tissue T1, and target constraint TC9 = 78 Gy being selected for target tissue T2.

In some embodiments, a computer simulation may be performed to create a dose volume histogram, which can be used to determine and/or to verify whether a particular target constraint has an effect of pushing up or lowering a radiation dose, as is known in the art. In some cases, a dose volume histogram may be presented in a form of a curve, which shows how much volume is below a certain radiation dose. Computer products for determining dose volume histograms are commercially available and are well known in the art. For example, Eclipse™ Treatment Planning System (Varian Medical Systems, Inc., Palo Alto, CA, USA) may be used to calculate dose volume histograms.

Next, a NTC curve is used to determine NTC values (associated with respective target tissues T1, T2) for the healthy tissue H1. As shown in **FIG. 3C,** based on the first distance d1 between the healthy tissue H1 and the target tissue T1, a first value f1 = 0.1 is determined using the NTC curve. Similarly, based on the distance d2 between the healthy tissue H2 and the target tissue T1, a second value f2 = 0.5 is determined using the NTC curve. Since the NTC curve is presented as normalized, or relative values, the actual NTC value at the healthy tissue H1 associated with target tissue T1 may be determined by multiplying **f1** with the selected TC value for the target tissue T1 (which is TC3 = 72 Gy in the example). As such, the first NTC value at the healthy tissue H1 is f1 x TC3 = 0.1 x 72 Gy = 7.2 Gy. Similarly, the NTC value at the healthy tissue H1 associated with target tissue T2 may be determined by multiplying f2 with the selected TC value for the target tissue T2 (which is TC9 = 78 Gy in the example). As such, the second NTC value at the healthy tissue H1 is f2 x TC9 = 0.5 x 78 Gy = 39 Gy. In alternative embodiments, instead of using a normalized NTC curve, two NTC curves that correspond to respective TC values selected for the target tissues T1, T2 may be used. For example, a first NTC curve may be determined by multiplying all values in a normalized NTC curve by the first selected TC for target tissue T1 (which is TC3 = 72 Gy in the example). Similarly, a second NTC curve may be determined by multiplying all values in a normalized NTC curve by the second selected TC for target tissue T2 (which is TC9 = 78 Gy in the example). In such cases, the first NTC value associated with the first target tissue T1 for the healthy tissue H1 can be determined directly from the first NTC curve, and the second NTC value associated with the second target tissue T1 for the healthy tissue H1 can be determined directly from the second NTC curve.

After the NTC values (7.2 Gy and 39 Gy in the example) have been determined for the healthy tissue H1, the highest NTC value is then selected as the constraint for the healthy tissue H1. In the example, this corresponds to the NTC value = 39 Gy being selected for the healthy tissue H1. As illustrated in the above embodiments, multiple tissue targets with different optimization objectives may be accomplished using NTC.

It should be noted that although the above example has been described with reference to two target tissues T1, T2, in other embodiments, the same technique may be applied using more than two target tissues. For example, in some embodiments, ten target tissue points/regions may be inputted in the mathematical model. In such cases, ten NTC values will be calculated for each healthy tissue H, and the constraint for each healthy tissue H is determined by selecting the highest NTC value among all of the NTC values. Also, in other embodiments, instead of determining-NTC value for one healthy tissue H1, the same technique may be applied to determine NTC values for more than one healthy tissue H.

In one of the above examples, the NTC normalized value 1.0 (or 100%) is selected to correspond to the lowest upper constraint set to a specific target. In some cases, if no upper constraint is set to the target, the level 1.0 (or 100%) may be selected to be 1.05 (or another value larger than 1.0) times the highest lower constraint set to the specific target.

In some embodiments, use of NTC may limit radiation dose level in healthy tissue, and may remove possible "hot spots", e.g. regions of high dose, in the healthy tissue. In some cases, use of NTC in accordance with embodiments described herein allows a treatment plan to be determined efficiently, and may eliminate the need to perform an iterative process of drawing new artificial structures that represent normal tissues.

In some embodiments, NTC can be used to perform beam angle optimization, which may be a part of a treatment planning. In beam angle optimization, different beam angles are determined such that the PTV is treated while minimizing injury to healthy tissue and hotspots. In other embodiments, instead of, or in addition to, performing beam angle optimization, NTC can be used to perform fluence optimization, which may also be a part of a treatment planning. Fluence optimization is a process in which a radiation flux, including intensity, form, energy, and modality, to patient from a plurality of radiation beams is optimized to minimize a cost function, which may include dose-based and treatment-based objectives. For example, in some embodiments, fluence optimization may be performed by considering a plurality of two dimensional images of a portion of a patient obtained using radiation delivered from different angles. The images may be used to obtain radiation absorption information, which in tum, may be used to confirm or determine an optimized set of radiation beams that will provide a desired dose to a target region.

In other embodiments, NTC may also be used for obtaining sharp gradients in the dose around targets in stereotactic radiotherapy (SRT) or stereotactic radiosurgery (SRS) treatments. For example, a suitably shaped NTC, when used in beam angle optimization and fluence optimization, may result in a treatment plan that causes a dose around the target be pushed down to thereby prevent delivery of dose to healthy tissue. In particular, NTC provides a static spatially-variant optimization objective that can be used to accomplish high dose conformity in stereotactic-type treatments. In some embodiments, the objective function may be a combination of objectives, such as, $f\_obj(d) = f\_obj\_1(d) + ... f\_obj\_n(d)$. In such cases, the optimization is "static" because the objective does not change according to dose, but for each point in the normal tissue volume, the objective is the same during an optimization. The optimization is also "spatially-variant" because the objective may be different for different spatial points.

In the above embodiments, the NTC technique is described with reference to an exponential model for modeling distance from target. However, the scope of the invention should not be so limited. In other embodiments, the NTC may use a quadratic objective for dose. For example, the following quadratic constraint may be used: $f\_obj(d) = w * (d-val)^2$, if d > val, and $f\_obj(d) = 0$, if d <= val, where "w" represents the weight imposed to the quadratic constraint, "d" represents the actual, calculated dose value and "val" represents the constraining dose value. Also, in further embodiments, instead of quadratic dose and exponential model for distance to the targets, the NTC may use a target shape, a body shape, and static spatially-variant dose objective.

Computer System Architecture

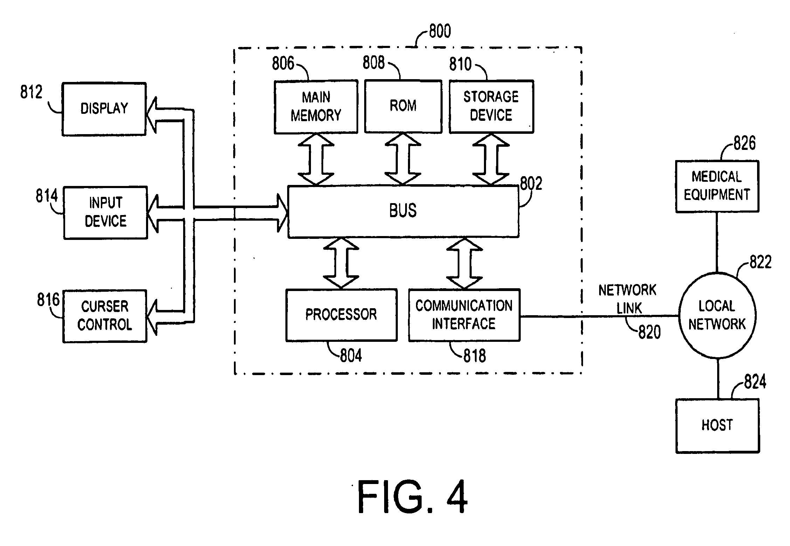

**FIG. 4** is a block diagram illustrating an embodiment of a computer system 800 that can be used to perform various functions described herein. In some embodiments, the computer system 800 can be used to calculate NTC value(s) based on various parameter values. In other embodiments, the computer system 800 may also be used to perform beam angle optimization and/or fluence optimization using embodiments of the NTC technique described herein.

Computer system 800 includes a bus 802 or other communication mechanism for communicating information, and a processor 804 coupled with the bus 802 for processing information. The processor 804 may be an example of the processor 54, or alternatively, an example of a component of the processor 54, of **FIG. 1.** The computer system 800 also includes a main memory 806, such as a random access memory (RAM) or other dynamic storage device, coupled to the bus 802 for storing information and instructions to be executed by the processor 804. The main memory 806 also may be used for storing temporary variables or other intermediate information during execution of instructions to be executed by the processor 804. The computer system 800 further includes a read only memory (ROM) 808 or other static storage device coupled to the bus 802 for storing static information and instructions for the processor 804. A data storage device 810, such as a magnetic disk or optical disk, is provided and coupled to the bus 802 for storing information and instructions.

The computer system 800 may be coupled via the bus 802 to a display 87, such as a cathode ray tube (CRT), for displaying information to a user. An input device 814, including alphanumeric and other keys, is coupled to the bus 802 for communicating information and command selections to processor 804. Another type of user input device is cursor control 816, such as a mouse, a trackball, or cursor direction keys for communicating direction information and command selections to processor 804 and for controlling cursor movement on display 87. This input device typically has two degrees of freedom in two axes, a first axis (e.g., x) and a second axis (e.g., y), that allows the device to specify positions in a plane.

In some embodiments, the computer system 800 can be used to perform various functions described herein. According to some embodiments, such use is provided by computer system 800 in response to processor 804 executing one or more sequences of one or more instructions contained in the main memory 806. Those skilled in the art will know how to prepare such instructions based on the functions and methods described herein. Such instructions may be read into the main memory 806 from another computer-readable medium, such as storage device 810. Execution of the sequences of instructions contained in the main memory 806 causes the processor 804 to perform the process steps described herein. One or more processors in a multi-processing arrangement may also be employed to execute the sequences of instructions contained in the main memory 806. In alternative embodiments, hard-wired circuitry may be used in place of or in combination with software instructions to implement the invention. Thus, embodiments of the invention are not limited to any specific combination of hardware circuitry and software.

The term "computer-readable medium" as used herein refers to any medium that participates in providing instructions to the processor 804 for execution. Such a medium may take many forms, including but not limited to, non-volatile media, volatile media, and transmission media. Non-volatile media includes, for example, optical or magnetic disks, such as the storage device 810. Volatile media includes dynamic memory, such as the main memory 806. Transmission media includes coaxial cables, copper wire and fiber optics, including the wires that comprise the bus 802. Transmission media can also take the form of acoustic or light waves, such as those generated during radio wave and infrared data communications.

Common forms of computer-readable media include, for example, a floppy disk, a flexible disk, hard disk, magnetic tape, or any other magnetic medium, a CD-ROM, any other optical medium, punch cards, paper tape, any other physical medium with patterns of holes, a RAM, a PROM, and EPROM, a FLASH-EPROM, any other memory chip or cartridge, a carrier wave as described hereinafter, or any other medium from which a computer can read.

Various forms of computer-readable media may be involved in carrying one or more sequences of one or more instructions to the processor 804 for execution. For example, the instructions may initially be carried on a magnetic disk of a remote computer. The remote computer can load the instructions into its dynamic memory and send the instructions over a telephone line using a modem. A modem local to the computer system 800 can receive the data on the telephone line and use an infrared transmitter to convert the data to an infrared signal. An infrared detector coupled to the bus 802 can receive the data carried in the infrared signal and place the data on the bus 802. The bus 802 carries the data to the main memory 806, from which the processor 804 retrieves and executes the instructions. The instructions received by the main memory 806 may optionally be stored on the storage device 810 either before or after execution by the processor 804.

The computer system 800 also includes a communication interface 818 coupled to the bus 802. The communication interface 818 provides a two-way data communication coupling to a network link 820 that is connected to a local network 822. For example, the communication interface 818 may be an integrated services digital network (ISDN) card or a modem to provide a data communication connection to a corresponding type of telephone line. As another example, the communication interface 818 may be a local area network (LAN) card to provide a data communication connection to a compatible LAN. Wireless links may also be implemented. In any such implementation, the communication interface 818 sends and receives electrical, electromagnetic or optical signals that carry data streams representing various types of information.

The network link 820 typically provides data communication through one or more networks to other devices. For example, the network link 820 may provide a connection through local network 822 to a host computer 824 or to equipment 826, such as any of the devices herein (e.g., device 166, system 10, patient support system 200, etc.), or a switch operatively coupled to any of the devices described herein. The data streams transported over the network link 820 can comprise electrical, electromagnetic or optical signals. The signals through the various networks and the signals on the network link 820 and through the communication interface 818, which carry data to and from the computer system 800, are exemplary forms of carrier waves transporting the information. The computer system 800 can send messages and receive data, including program code, through the network(s), the network link 820, and the communication interface 818.

Although particular embodiments have been shown and described, it will be understood that it is not intended to limit the claimed inventions, and it will be obvious to those skilled in the art that various changes and modifications may be made without departing from the spirit and scope of the application. For example, in other embodiments, the system 10 may not include one or more of the components described herein. Also, the operations performed by the processor 54 can be performed by any combination of hardware and software, and should not be limited to particular embodiments comprising a particular definition of "processor." The specification and drawings are, accordingly, to be regarded in an illustrative rather than restrictive sense. The present inventions are intended to cover alternatives, modifications, and equivalents, which may be included within the scope of the present inventions as defined by the claims.

## Claims

1.  A method for use to determine or evaluate a radiation treatment plan, comprising:

    determining a spatially variable constraint; and
    imposing the spatially variable constraint on a healthy tissue (H);
    **characterized in that** the spatially variable constraint is variable based at least in part on a distance (d) from a target tissue (T), and is represented by a function that decreases exponentially, and that the method further comprises using the spatially variable constraint to determine or evaluate the radiation treatment plan.

2.  The method of claim 1, wherein the distance is between the healthy tissue (H) and a boundary of the target tissue (T).

3.  The method of claim 1, wherein the target tissue (T) is a point located in or on a Planning Target Volume.

4.  The method of claim 1, wherein the target tissue (T) is a point located in or on a Clinical Target Volume.

5.  The method of claim 1, further comprising predicting a result of a radiation treatment based on the imposed spatially variable constraint.

6.  The method of claim 1, further comprising determining a treatment plan based on the imposed spatially variable constraint.

7.  The method of claim 1, further comprising determining a value of the spatially variable constraint using a process that includes:

    determining a first position of a first target tissue (T1), the first target tissue (T1) being the target tissue (T);
    determining a first target constraint value;
    calculating a first value based on the first target constraint value;
    determining a second position of a second target tissue (T2);
    determining a second target constraint value;
    calculating a second value based on the second target constraint value; and
    selecting a maximum of the first and second values.

8.  The method of claim 7, wherein the first target constraint value is determined by:

    determining a plurality of target constraint values;
    determining a subset of the target constraint values having increasing radiation dose associated therewith; and
    selecting a minimum value from the subset as the first target constraint.

9.  The method of claim 1, wherein the function comprises:

$$f(x) = \begin{cases} f_o e^{-k(x-x_{start})} + f_\infty(1 - e^{-k(x-x_{start})}), & x \geq x_{start} \\ f_o, & x \leq x_{start} \end{cases}$$

**10.** The method of claim 1, wherein the spatially variable constraint is associated with a limit of radiation dose to be received by the healthy tissue (H).

**11.** The method of claim 1, wherein the healthy tissue (H) comprises a mathematical model of a real healthy tissue.

**12.** The method of claim 1, further comprising:

determining a first position of a first healthy tissue (H1), the first healthy tissue (H1) being the healthy tissue (H);
determining a second position of a second healthy tissue (H2); and
imposing the spatially variable constraint on the second healthy tissue (H2);
wherein the acts of imposing the spatially variable constraint comprises:

imposing a first constraint on the first healthy tissue (H1) based on the determined first position; and
imposing a second constraint on the second healthy tissue (H2) based on the determined second position, wherein the first constraint and the second constraint have different values.

**13.** The method of claim 12, wherein the first healthy tissue (H1) is closer to the target tissue (T) than the second healthy tissue (H2), and the value of the first constraint is higher than the value of the second constraint.

**14.** The method of claim 12, wherein the distance (d) is between the first healthy tissue (H1) and a boundary of the target tissue (T), the method further comprising determining the value of the first constraint based at least in part on the distance (d).

**15.** The method of claim 12, further comprising predicting a result of a radiation treatment based on the first and second constraints.

**16.** The method of claim 12, further comprising determining a treatment plan based on the first and second constraints.

**17.** The method of claim 12, further comprising determining a value of the first constraint based on positions of at least two target tissues (T).

**18.** The method of claim 12, wherein the values of the first and second constraints lie on a curve in which x values represent distances from a boundary of the target tissue (T), and y values represent constraint values, the curve having a decreasing profile.

**19.** A system (10) for determining or evaluating a radiation treatment plan, comprising:

a processor configured for
determining a spatially variable constraint; and
imposing the spatially variable constraint on a healthy tissue (H);
**characterized in that** the spatially variable constraint is variable based at least in part on a distance (d) from a target tissue (T), and is represented by a function that decreases exponentially, and that the processor is further configured for using the spatially variable constraint to determine or evaluate the radiation treatment plan.

**Patentansprüche**

**1.** Ein Verfahren zur Verwendung zum Ermitteln oder Beurteilen eines Strahlenbehandlungsplans, aufweisend:

Ermitteln einer räumlich variablen Beschränkung, und
Auferlegen der räumlich variablen Beschränkung auf ein gesundes Gewebe (H),

**dadurch gekennzeichnet, dass** die räumlich variable Beschränkung wenigstens teilweise auf einer Distanz (d) von einem zielgewebe (T) aus basierend variabel ist und durch eine Funktion repräsentiert ist, welche exponentiell abnimmt, und dass das Verfahren ferner aufweist: Verwenden der räumlich variablen Beschränkung, um den Strahlenbehandlungsplan zu ermitteln oder beurteilen.

2. Das Verfahren gemäß Anspruch 1, wobei die Distanz zwischen dem gesunden Gewebe (H) und einer Grenze des Zielgewebes (T) liegt.

3. Das Verfahren gemäß Anspruch 1, wobei das Zielgewebe (T) ein Punkt ist, welcher sich in oder an einem Planzielvolumen befindet.

4. Das Verfahren gemäß Anspruch 1, wobei das Zielgewebe (T) ein Punkt ist, welcher sich in oder an einem klinischen Zielvolumen befindet.

5. Das Verfahren gemäß Anspruch 1, ferner aufweisend:

Prognostizieren eines Ergebnisses einer Strahlenbehandlung basierend auf der auferlegten räumlich variablen Beschränkung.

6. Das Verfahren gemäß Anspruch 1, ferner aufweisend: Ermitteln eines Behandlungsplans basierend auf der auferlegten räumlich variablen Beschränkung.

7. Das Verfahren gemäß Anspruch 1, ferner aufweisend: Ermitteln eines Werts der räumlich variablen Beschränkung unter Verwendung eines Vorgangs, welcher aufweist:

Ermitteln einer ersten Position eines ersten Zielgewebes (T1), wobei das erste Zielgewebe (T1) das Zielgewebe (T) ist,
Ermitteln eines ersten Ziel-Beschränkungswerts,
Berechnen eines ersten Werts basierend auf dem ersten Ziel-Beschränkungswert,
Ermitteln einer zweiten Position eines zweiten Zielgewebes (T2),
Ermitteln eines zweiten Ziel-Beschränkungswerts,
Berechnen eines zweiten Werts basierend auf dem zweiten Ziel-Beschränkungswert, und
Auswählen eines Maximums von dem ersten und dem zweiten Wert.

8. Das Verfahren gemäß Anspruch 7, wobei der erste Ziel-Beschränkungswert ermittelt wird durch:

Ermitteln einer Mehrzahl von Ziel-Beschränkungswerten,
Ermitteln einer Teilmenge von den Ziel-Beschränkungswerten, mit welchen eine zunehmende Strahlendosis verbunden ist, und
Auswählen eines Minimalwerts aus der Teilmenge als den ersten Ziel-Beschränkungswert.

9. Das Verfahren gemäß Anspruch 1, wobei die Funktion aufweist:

$$f(x) = \begin{cases} f_0 e^{-k(x-x_{Start})} + f_\infty\left(1 - e^{-k(x-x_{Start})}\right), & x \geq x_{Start} \\ f_0, & x \geq x_{Start} \end{cases}$$

10. Das Verfahren gemäß Anspruch 1, wobei die räumlich variable Beschränkung im Zusammenhang mit einem Höchstmaß an Strahlendosis steht, welche das gesunde Gewebe (H) erhalten wird.

11. Das Verfahren gemäß Anspruch 1, wobei das gesunde Gewebe (H) ein mathematisches Modell eines realen gesunden Gewebes aufweist.

12. Das Verfahren gemäß Anspruch 1, ferner aufweisend:

Ermitteln einer ersten Position eines ersten gesunden Gewebes (H1), wobei das erste gesunde Gewebe (H1) das gesunde Gewebe (H) ist,

Ermitteln einer zweiten Position eines zweiten gesunden Gewebes (H2), und
Auferlegen der räumlich variablen Beschränkung auf das zweite gesunde Gewebe (H2),
wobei die Vorgänge des Auferlegens der räumlich variablen Beschränkung aufweisen:

Auferlegen einer ersten Beschränkung auf das erste gesunde Gewebe (H1) basierend auf der ermittelten ersten Position, und
Auferlegen einer zweiten Beschränkung auf das zweite gesunde Gewebe (H2) basierend auf der ermittelten zweiten Position, wobei die erste Beschränkung und die zweite Beschränkung unterschiedliche Werte haben.

13. Das Verfahren gemäß Anspruch 12, wobei das erste gesunde Gewebe (H1) näher an dem Zielgewebe (T) ist als das zweite gesunde Gewebe (H2) und wobei der Wert der ersten Beschränkung höher ist als der Wert der zweiten Beschränkung.

14. Das Verfahren gemäß Anspruch 12, wobei die Distanz (d) zwischen dem ersten gesunden Gewebe (H1) und einer Grenze des Zielgewebes (T) liegt, wobei das Verfahren ferner aufweist:

Ermitteln des Werts der ersten Beschränkung wenigstens teilweise basierend auf der Distanz (d).

15. Das Verfahren gemäß Anspruch 12, ferner aufweisend:

Prognostizieren eines Ergebnisses einer Strahlenbehandlung basierend auf der ersten und der zweiten Beschränkung.

16. Das Verfahren gemäß Anspruch 12, ferner aufweisend:

Ermitteln eines Behandlungsplans basierend auf der ersten und
der zweiten Beschränkung.

17. Das Verfahren gemäß Anspruch 12, ferner aufweisend:

Ermitteln eines Werts der ersten Beschränkung basierend auf Positionen von wenigstens zwei Zielgeweben (T).

18. Das Verfahren gemäß Anspruch 12, wobei die Werte der ersten und der zweiten Beschränkung auf einer Kurve liegen, wobei x- Werte Abstände von einer Grenze des Zielgewebes (T) darstellen und y-werte Beschränkungswerte darstellen, wobei die Kurve ein abnehmendes Profil aufweist.

19. Ein System (10) zum Ermitteln oder Beurteilen eines Strahlenbehandlungsplans, aufweisend:

einen Prozessor, welcher eingerichtet ist zum
Ermitteln einer räumlich variablen Beschränkung und
Auferlegen der räumlich variablen Beschränkung auf ein gesundes Gewebe (H),
**dadurch gekennzeichnet, dass** die räumlich variable Beschränkung wenigstens teilweise auf einer Distanz (d) von einem Zielgewebe (T) aus basierend variabel ist und durch eine Funktion repräsentiert ist, welche exponentiell abnimmt, und dass der Prozessor ferner eingerichtet ist zum verwenden der räumlich variablen Beschränkung, um den Strahlenbehandlungsplan zu ermitteln oder beurteilen.

**Revendications**

1. Procédé à utiliser pour déterminer ou évaluer un schéma de radiothérapie, comprenant :

la détermination d'une contrainte variable dans l'espace ; et
l'imposition de la contrainte variable dans l'espace sur un tissu sain (H) ;
**caractérisé en ce que** la contrainte variable dans l'espace est variable en se basant au moins partiellement sur une distance (d) depuis un tissu cible (T), et est représentée par une fonction qui décroît de façon exponentielle, et **en ce que** le procédé comprend en outre l'utilisation de la contrainte variable dans l'espace pour déterminer ou évaluer le schéma de radiothérapie.

**2.** Procédé selon la revendication 1, dans lequel la distance est comprise entre le tissu sain (H) et une limite du tissu cible (T).

**3.** Procédé selon la revendication 1, dans lequel le tissu cible (T) est un point situé dans ou sur un volume cible planifé.

**4.** Procédé selon la revendication 1, dans lequel le tissu cible (T) est un point situé dans ou sur un volume cible clinique.

**5.** Procédé selon la revendication 1, comprenant en outre la prévision d'un résultat d'une radiothérapie basée sur la contrainte variable dans l'espace imposée.

**6.** Procédé selon la revendication 1, comprenant en outre la détermination d'un schéma thérapeutique basée sur la contrainte variable dans l'espace imposée.

**7.** Procédé selon la revendication 1, comprenant en outre la détermination d'une valeur de la contrainte variable dans l'espace en utilisant un processus qui inclut :

la détermination d'une première position d'un premier tissu cible (T1), le premier tissu cible (T1) étant le tissu cible (T) ;
la détermination d'une première valeur de contrainte cible ;
le calcul d'une première valeur basé sur la première valeur de contrainte cible ;
la détermination d'une seconde position d'un second tissu cible (T2) ;
la détermination d'une seconde valeur de contrainte cible ;
le calcul d'une seconde valeur basé sur la seconde valeur de contrainte cible ; et
la sélection d'un maximum des première et seconde valeurs.

**8.** Procédé selon la revendication 7, dans lequel la première valeur de contrainte cible est déterminée en :

déterminant une pluralité de valeurs de contrainte cibles ;
déterminant un sous-ensemble des valeurs de contrainte cibles auquel est associée une dose croissante de rayonnement ; et
sélectionnant une valeur minimale à partir du sous-ensemble en tant que première contrainte cible.

**9.** Procédé selon la revendication 1, dans lequel la fonction comprend:

$$f(x) = \begin{cases} f_0 e^{-k(x-x_{start})} + f_\infty (1 - e^{-k(x-x_{start})}), & x \geq x_{start} \\ f_0, & x \leq x_{start} \end{cases}$$

**10.** Procédé selon la revendication 1, dans lequel la contrainte variable dans l'espace est associée à une limite de dose de rayonnement que le tissu sain (H) peut recevoir.

**11.** Procédé selon la revendication 1, dans lequel le tissu sain (H) comprend un modèle mathématique d'un tissu sain réel.

**12.** Procédé selon la revendication 1, comprenant en outre :

la détermination d'une première position d'un premier tissu sain (H1), le premier tissu sain (H1) étant le tissu sain (H) ;
la détermination d'une seconde position d'un second tissu sain (H2) ; et
l'imposition de la contrainte variable dans l'espace sur le second tissu sain (H2) ;
dans lequel les étapes consistant à imposer la contrainte variable dans l'espace comprennent :

l'imposition d'une première contrainte sur le premier tissu sain (H1) basée sur la première position déterminée ; et
l'imposition d'une seconde contrainte sur le second tissu sain (H2) basée sur la seconde position déterminée, dans lequel la première contrainte et la seconde contrainte présentent des valeurs différentes.

**13.** Procédé selon la revendication 12, dans lequel le premier tissu sain (H1) est plus proche du tissu cible (T) que le second tissu sain (H2), et la valeur de la première contrainte est supérieure à la valeur de la seconde contrainte.

**14.** Procédé selon la revendication 12, dans lequel la distance (d) est comprise entre le premier tissu sain (H1) et une limite du tissu cible (T), le procédé comprenant en outre la détermination de la valeur de la première contrainte en se basant au moins partiellement sur la distance (d).

**15.** Procédé selon la revendication 12, comprenant en outre la prévision d'un résultat d'une radiothérapie basée sur des première et seconde contraintes.

**16.** Procédé selon la revendication 12, comprenant en outre la détermination d'un schéma thérapeutique basée sur des première et seconde contraintes.

**17.** Procédé selon la revendication 12, comprenant en outre la détermination d'une valeur de la première contrainte basée sur les positions d'au moins deux tissus cibles (T).

**18.** Procédé selon la revendication 12, dans lequel les valeurs des première et seconde contraintes se trouvent sur une courbe sur laquelle les valeurs x représentent des distances à partir d'une limite du tissu cible (T), et les valeurs y représentent des valeurs de contrainte, la courbe présentant un profil décroissant.

**19.** Système (10) de détermination ou d'évaluation d'un schéma de radiothérapie, comprenant :

un processeur configuré pour :

déterminer une contrainte variable dans l'espace ; et
imposer la contrainte variable dans l'espace sur un tissu sain (H) ;
**caractérisé en ce que** la contrainte variable dans l'espace est variable en se basant au moins partiellement sur une distance (d) depuis un tissu cible (T), et est représentée par une fonction qui décroît de façon exponentielle, et **en ce que** le processeur est configuré en outre pour utiliser la contrainte variable dans l'espace afin de déterminer ou d'évaluer le schéma de radiothérapie.

FIG. 1

Normal Tissue Constraint

FIG. 2

**FIG. 3A**

T1)

| Constraint | TC1 | TC2 | TC3 | TC4 | TC5 |
|---|---|---|---|---|---|
| Value | 62 | 70 | 72 | 85 | 83 |
| Radiation | up | up | down | down | down |

T2)

| Constraint | TC6 | TC7 | TC8 | TC9 | TC10 |
|---|---|---|---|---|---|
| Value | 60 | 62 | 70 | 78 | 79 |
| Radiation | up | up | up | down | down |

**FIG. 3B**

**FIG. 3C**

FIG. 4

**EP 2 038 010 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2006067469 A1
- US 3987281 A
- US 03332701 A
- US 68757303 A